# FASCICULE DE BREVET EUROPEEN

(11) **EP 1 493 379 B1**
(45) Date de publication et mention de la délivrance du brevet: **21.03.2007**
(21) Numéro de dépôt: 04291478.8
(22) Date de dépôt: 11.06.2004
(51) Int. Cl.: A61B 1/12

(54) **Dispositif d'exploitation amovible pour sonde endoscopique souple à vocation médicale**
Abnehmbare Steuervorrichtung für endoskopische Sonde für medizinische Anwendungen
Removable switching device for endoscopic probe for medical use

(30) Priorité: 04.07.2003 FR 0308223
(43) Date de publication de la demande: 05.01.2005
(73) Titulaire: Tokendo, 13600 La Ciotat (FR)
(72) Inventeur: Rovegno, Jean, 13600 La Ciotat (FR)
(74) Mandataire: de Roquemaurel, Bruno

(56) Documents cités:
- WO-A-93/25139
- DE-C- 19 721 030
- US-A- 3 830 225

## Description

La présente invention concerne le domaine des sondes endoscopiques souples à vocation médicale du type fibroscope ou vidéoendoscope, disposant d'un canal opératoire, d'une entrée instrumentation et d'une commande d'aspiration.

Elle s'applique notamment au domaine de l'endoscopie médicale, et plus particulièrement à la bronchoscopie.
On désigne généralement par le terme "endoscopie" un examen visuel réalisé à l'intérieur d'une cavité obscure à l'aide d'un "endoscope", d'un "fibroscope", ou d'un "vidéoendoscope".

On désigne généralement par le terme "fibroscope" une sonde endoscopique souple qui, introduite dans une cavité obscure, permet à un utilisateur d'une part d'observer dans un oculaire une cible située à l'intérieur de la cavité et d'autre part de modifier l'orientation de l'extrémité distale de la sonde à l'intérieur de la cavité. Pour ce faire, un fibroscope intègre par nature un dispositif optique, un dispositif d'illumination et un dispositif mécanique. Le dispositif mécanique d'un fibroscope est constitué d'un béquillage distal articulé, d'une poignée de commande et de quatre câbles logés à l'intérieur d'une gaine cylindrique souple reliant la poignée de commande au béquillage distal articulé. La poignée du fibroscope dispose de deux dispositifs commandés manuellement et agissant l'un sur la paire de câbles actionnant l'orientation du béquillage dans un plan et l'autre sur la paire de câbles actionnant l'orientation du béquillage dans un plan perpendiculaire au précédent. Le dispositif optique d'un fibroscope est constitué d'un objectif logé dans la face distale d'un embout solidaire de l'extrémité distale du béquillage, d'un système de transport optique de l'image délivrée par l'objectif distal, le système de transport étant constitué d'un faisceau souple de fibres optiques ordonnées cheminant successivement dans le béquillage articulé, dans la gaine souple puis dans la poignée et reliant l'oculaire à l'objectif distal, et d'un oculaire à réglage dioptrique solidaire de la poignée de commande et permettant à l'utilisateur d'observer l'image magnifiée de la cible située devant l'objectif distal du fibroscope et transmise par le système de transport optique d'image. Le dispositif d'illumination d'un fibroscope est constitué d'un faisceau de fibres optiques non ordonnées cheminant successivement dans le béquillage articulé, dans la gaine souple, dans la poignée, puis dans un câble ombilical solidaire de la poignée de commande. L'extrémité distale du faisceau de fibres, logée dans la face distale de l'embout solidaire de l'extrémité distale du béquillage articulé, illumine la cible observée quand son extrémité proximale, logée dans un embout d'éclairage intégré dans le dispositif de connexion constituant l'extrémité proximale du câble ombilical, est reliée à un générateur de lumière. L'exploitation du fibroscope décrit ci-dessus nécessite dans ces conditions l'utilisation conjointe d'un générateur de lumière.

On désigne généralement par le terme "vidéoendoscope" une sonde endoscopique souple qui, introduite dans une cavité obscure, permet à un utilisateur d'une part d'observer sur l'écran d'un moniteur vidéo une cible située à l'intérieur de la cavité et d'autre part de modifier l'orientation de l'extrémité distale de la sonde à l'intérieur de la cavité. Pour ce faire un vidéoendoscope comprend par nature un dispositif d'imagerie, un dispositif d'illumination et un dispositif mécanique. Le dispositif d'illumination et le dispositif mécanique d'un vidéoendoscope sont en tous points identiques aux dispositifs de même nature intégrés dans un fibroscope et décrits ci-dessus. Le dispositif d'imagerie d'un vidéoendoscope comprend généralement les éléments suivants :
- un dispositif optoélectronique logé dans l'embout distal solidaire de l'extrémité distale du béquillage articulé et comprenant un objectif logé dans la face distale dudit embout, un capteur CCD couleur sur le substrat photoélectrique duquel se forme l'image réelle de la cible observée, délivrée par l'objectif et un microcircuit d'interface destiné à corriger les signaux électriques reçus ou générés par le capteur CCD ;
- un câble électrique multiconducteurs cheminant successivement dans le béquillage articulé, dans la gaine souple, dans la poignée de commande, puis dans le câble ombilical solidaire de la poignée de commande, l'extrémité distale dudit câble étant électriquement solidaire du microcircuit d'interface du dispositif optoélectronique distal, tandis que son extrémité proximale est électriquement solidaire d'une embase de connexion multibroches intégrée dans le dispositif de connexion constituant l'extrémité proximale du câble ombilical ;
- un processeur vidéo assurant la synchronisation et l'alimentation électrique du dispositif optoélectronique distal, le traitement du signal électrique généré par le dispositif optoélectronique, et délivrant un signal vidéo directement exploitable sur un moniteur couleur ; le processeur vidéo disposant d'un panneau de touches de commande permettant à l'utilisateur de régler les paramètres de l'image vidéo, d'un connecteur de sortie vidéo destiné à être relié à un moniteur vidéo, et d'un connecteur multibroches destiné à être relié à une embase de connexion multibroches intégrée dans le dispositif de connexion constituant l'extrémité proximale du câble ombilical du vidéoendoscope.

L'exploitation du vidéoendoscope décrit ci-dessus nécessite dans ces conditions l'utilisation conjointe d'un moniteur vidéo et d'un générateur de lumière, le générateur de lumière étant souvent associé dans un même coffret au processeur vidéo du vidéoendoscope.

Les sondes endoscopiques souples, du type fibroscope ou du type vidéoendoscope, concernées par la présente invention et destinées notamment à des applications médicales telles que la bronchoscopie, disposent en outre d'un canal opératoire constitué d'un tuyau cylindrique flexible cheminant successivement dans le béquillage articulé, dans la gaine cylindrique souple, puis dans la partie distale de la poignée de commande des sondes endoscopiques. Le canal opératoire, dont l'extrémité distale est située au niveau de la face distale de l'embout distal des sondes endoscopiques, permet de réaliser tout ou partie des opérations suivantes :
- introduction d'un instrument souple, tel que pince, bistouri électrique ou fibre laser, permettant à l'utilisateur d'intervenir physiquement sur la cible située devant l'objectif distal de la sonde endoscopique souple ;
- aspiration au niveau de la cible de débris de tissus résultant des interventions évoquées ci-dessus ;
- diffusion d'un produit médicamenteux liquide au niveau de la cible.

La mise en oeuvre de ces diverses opérations nécessite l'intégration dans les sondes endoscopiques souples dédiées à ce type d'applications, d'un dispositif d'exploitation comprenant les éléments suivants :
- le canal opératoire flexible évoqué ci-dessus ;
- une entrée aspiration solidaire de la poignée de commande et destinée à être raccordée par un tuyau flexible externe à une pompe d'aspiration ;
- un robinet de commande généralement solidaire de la poignée de commande et permettant à l'utilisateur de relier une entrée d'aspiration à l'extrémité proximale du canal opératoire ;
- une entrée d'instrument solidaire de la poignée de commande et directement reliée à l'extrémité proximale du canal opératoire, la entrée d'instrument disposant généralement d'un capuchon amovible en silicone assurant l'étanchéité de la entrée et permettant également l'introduction soit d'un instrument flexible soit de l'embout conique d'une seringue contenant un produit destiné à être injecté dans le canal opératoire.

L'architecture du dispositif opératoire des sondes endoscopiques souples permettant la mise en oeuvre des opérations évoquées ci-dessus doit répondre simultanément à des contraintes ergonomiques et à des contraintes de désinfection.

Les contraintes ergonomiques concernent l'accès aux fonctions de "commande d'aspiration" et d'introduction d'instrument(s) qui doivent être disposées sur la poignée de commande de façon à faciliter le travail de l'opérateur.

Quant aux contraintes de désinfection, elles concernent aussi bien les éléments fixes du dispositif opératoire, intégrés dans la sonde endoscopique que les éléments amovibles externes dudit dispositif. Lesdits éléments amovibles, tels que robinet de commande d'aspiration et capuchon d'étanchéité, doivent soit être du type à usage unique, soit être démontables afin de pouvoir être nettoyés par brossage avant d'être désinfectés par immersion dans un bain désinfectant ou, mieux, par passage à l'autoclave. Quant aux éléments fixes du dispositif opératoire, tels que le canal opératoire et les divers tuyaux de liaison fixement intégrés dans la poignée de commande, ils doivent pouvoir être aisément nettoyés à l'aide de brosses cylindriques avant que la sonde endoscopique soit entièrement immergée dans un bain de désinfection.

Les sondes endoscopiques souples à vocation médicale permettant la mise en oeuvre d'une commande d'aspiration et d'une entrée instrumentation relèvent en pratique de deux types d'architecture.

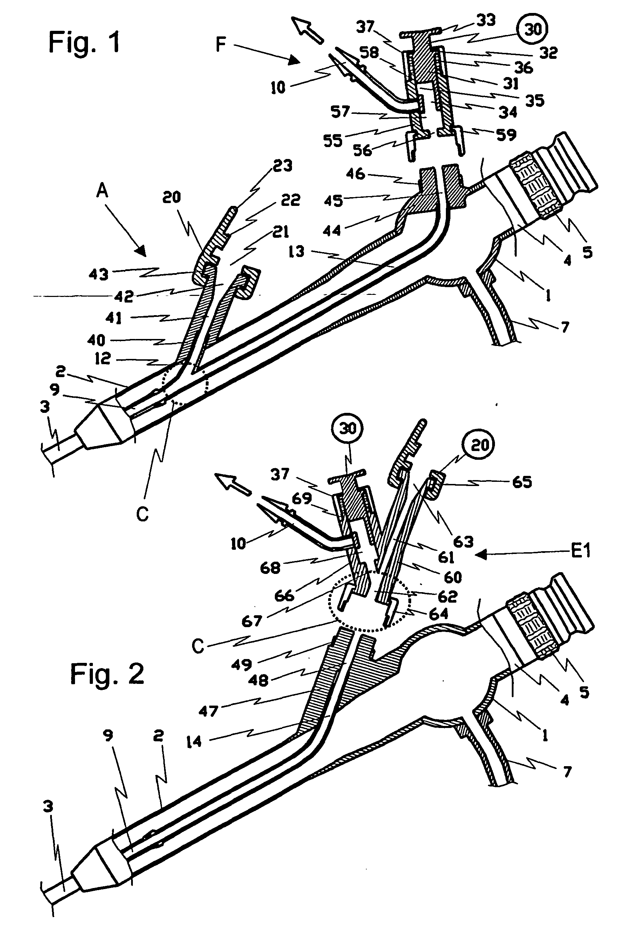

La première architecture couramment adoptée dans les sondes endoscopiques souples permettant la mise en oeuvre d'une commande d'aspiration et d'une entrée instrumentation concerne des sondes telles que celle représentée à la figure 1, dont la poignée de commande 1 comporte deux accès distincts 41 et 45 au canal opératoire 9 reliés par une tubulure 12, 13 intégrée dans la poignée 1. Le premier de ces accès 41, disposé au niveau de l'extrémité distale de la poignée 1 de commande, concerne l'entrée d'instrument. Le second accès 45, disposé au niveau de la partie proximale de la poignée 1, est quant à lui équipé d'un ensemble d'aspiration F externe amovible, et regroupe la commande d'aspiration 30 et une tubulure d'aspiration 10 comprenant un embout de raccordement à une pompe d'aspiration externe. Un tel dispositif d'exploitation a été décrit par la société japonaise OLYMPUS dans les brevets US 5299561 et US 5840015. Un autre dispositif d'exploitation de ce type a été décrit par la société japonaise OLYMPUS dans les brevets US 5257773 et US 5322263. De tels dispositifs ont par ailleurs été mis en oeuvre dans les fibroscopes de la série FB et les vidéoendoscopes de la série EB fabriqués par la société japonaise PENTAX, ainsi que les fibroscopes des séries 40 et 160 fabriqués par la société japonaise OLYMPUS. L'implantation de l'entrée d'instrument 41 et de la commande d'aspiration 30 du dispositif d'aspiration F, aux extrémités de la poignée de commande 1 des sondes endoscopiques souples, relevant de ce type d'architecture présente un avantage indéniable en matière d'ergonomie.

Il est en effet plus ergonomique d'utiliser une main pour tenir la poignée 1 de la sonde et éventuellement manipuler la commande d'aspiration 30 de l'ensemble d'aspiration F et d'utiliser l'autre main pour manipuler l'instrument opératoire introduit dans l'entrée d'instrument 41 qui est éloignée de la commande d'aspiration 30.

Par contre, l'intégration dans la poignée de commande 1 d'une tubulure interne 12 et 13 reliant les deux entrées 41 et 45 au canal opératoire 9 constitue un handicap certain en matière de facilité de nettoyage et de désinfection.

Il est en effet difficile, voir impossible de démonter cette tubulure interne 12, 13 de la poignée pour la nettoyer. En ce cas, le nettoyage est généralement réalisé à l'intérieur même de la poignée de commande et ce au risque de laisser des impuretés au niveau de l'embranchement du Y constitué par les tubulures 13/12/9, endroit particulièrement propice à l'accumulation de débris de tissus.

La seconde de ces architectures concerne des sondes endoscopiques, telle que celle décrite à la figure 2, dont la poignée de commande dispose d'un seul et unique accès au canal opératoire, cet accès étant équipé d'un dispositif d'exploitation E1 externe, totalement ou partiellement amovible suivant les modèles, et regroupant l'entrée d'instrument, la commande d'aspiration et l'embout de raccordement à une pompe d'aspiration externe. Différents dispositifs d'exploitation de ce type ont été décrits par la société japonaise OLYMPUS dans les brevets US 4198958, US 4469090, US 4561428, US 4736732 et US 4794913. De tels dispositifs ont par ailleurs été mis en oeuvre dans les bronchoscopes de la série FBS fabriqués par la société japonaise MACHIDA et dans les bronchoscopes de la série 20 fabriqués par la société japonaise OLYMPUS. Un autre dispositif d'exploitation de ce type a été décrit dans le brevet US 3 830 225.

L'accès direct au canal opératoire qui caractérise les sondes endoscopiques souples relevant de ce type d'architecture présente un avantage indéniable en matière de facilité de nettoyage et de désinfection, car le dispositif d'exploitation externe peut être démonté aisément de la poignée de l'endoscope.

Par contre, en raison de la proximité de l'entrée d'instrument et de la commande d'aspiration, ce dispositif d'exploitation E1 amovible monobloc est peu ergonomique à utiliser. Dans le brevet US 3 830 225, la commande d'aspiration a été montée à l'extrémité d'un conduit souple.

La présente invention a pour but de réaliser un dispositif d'exploitation amovible pour sonde endoscopique souple à vocation préférentiellement médicale et disposant d'un seul et unique accès au canal opératoire 9, ce dispositif d'exploitation devant être à la fois démontable facilement, ergonomique à manipuler, et répondre aux contraintes de désinfection exigées par les utilisateurs.

Cet objectif est atteint par la prévision d'une sonde endoscopique selon la revendication 1.

Selon l'invention, ce dispositif d'exploitation comprend un conduit de liaison mettant en communication le conduit d'aspiration avec le conduit d'insertion, et dont la longueur est telle que lorsque le dispositif d'exploitation est fixé sur une poignée de sonde endoscopique, le moyen de commande d'aspiration soit positionné à proximité de moyens de commande de ladite poignée situés en position proximale de ladite poignée.

Ce dispositif d'exploitation est dès lors à la fois démontable de la poignée 1 d'endoscope pour être par exemple stérilisé, et également ergonomique à utiliser car la commande d'aspiration 30 se trouve suffisamment proche des moyens de commande de l'endoscope pour être manipulable avec la même main que ces derniers.

Avantageusement, la longueur du conduit de liaison est de l'ordre de l'épaisseur de plusieurs doigts d'une main.

De préférence, les moyens de connexion du conduit d'insertion sont conçus pour assurer la fixation amovible du dispositif d'exploitation sur la poignée.

Selon un mode de réalisation de l'invention, ce dispositif d'exploitation comprend un moyen de fixation supplémentaire situé au niveau de l'ensemble d'aspiration, conçu pour coopérer avec des moyens de fixation complémentaires prévus sur la partie proximale de la poignée.

De préférence, le conduit de liaison 16 est constitué en matériau rigide.

Selon un autre mode de réalisation de l'invention, le moyen de commande d'aspiration est un robinet démontable pouvant adopter au moins une configuration d'ouverture autorisant le passage de fluide entre le canal opératoire et une tubulure d'aspiration elle-même reliée à la pompe d'aspiration, et une configuration de fermeture interdisant le passage de fluide vers la tubulure d'aspiration.

Selon encore un autre mode de réalisation de l'invention, l'ensemble d'aspiration comporte une pièce cylindrique montée coulissante à l'intérieur du conduit d'aspiration, et comprenant un canal axial borgne et un orifice latéral reliant l'extérieur de la pièce cylindrique à l'intérieur du canal cylindrique axial borgne, la pièce cylindrique obturant en configuration de fermeture, l'entrée de la tubulure d'aspiration, l'orifice latéral étant en configuration d'ouverture, en regard de l'entrée de la tubulure d'aspiration.

De préférence, la pièce cylindrique est ramenée en configuration de fermeture par des moyens de rappel élastiques.

Selon encore un autre mode de réalisation de l'invention, le conduit d'aspiration de l'ensemble d'aspiration est relié sélectivement par le moyen de commande à une tubulure d'injection permettant l'injection sélective de fluides provenant de la tubulure d'injection vers le canal opératoire.

Avantageusement, le moyen d'insertion pour l'insertion d'instruments opératoires est doté d'un capuchon permettant l'obturation du conduit d'insertion.

De préférence, ce dispositif d'exploitation est réalisé essentiellement en matériau stérilisable à usage médical tel que de l'inox.

Avantageusement, ce dispositif d'exploitation est réalisé de manière à pouvoir être à usage unique.

L'invention concerne également un fibroscope et un vidéoendoscope, équipé du dispositif d'exploitation défini ci-avant

Un mode de réalisation préféré de l'invention sera décrit ci-après, à titre d'exemple non limitatif, avec référence aux dessins annexés dans lesquels :
La figure 1 brièvement présentée ci-dessus, représente, en coupe transversale, une poignée d'endoscope et son dispositif d'exploitation partiellement amovible, selon l'art antérieur.
La figure 2 brièvement présentée ci-dessus, représente, en coupe transversale, une poignée d'endoscope et son dispositif d'exploitation totalement amovible, selon l'art antérieur.
La figure 3 représente, en coupe transversale, une poignée d'endoscope et un dispositif d'exploitation selon l'invention.
La figure 4 montre, en coupe transversale, une variante selon l'invention du dispositif d'exploitation montré sur la figure 3.
La figure 5 représente, en vue de profil, une poignée de commande de sonde vidéoendoscopique équipée du dispositif d'exploitation selon l'invention.

La figure 1 illustre une poignée de commande 1 d'un fibroscope à vocation médicale, de l'art antérieur, disposant de deux accès distincts 41 et 45 au canal opératoire 9 et d'un dispositif d'exploitation externe également représentatif de l'état de l'art en la matière.

Comme toutes les sondes endoscopiques souples à vocation médicale équipées d'un canal opératoire, ce fibroscope dispose d'une poignée de commande 1 solidaire de l'extrémité distale d'un câble ombilical souple 7 et de l'extrémité proximale d'une gaine souple 3 servant notamment de logement au canal opératoire souple 9 dont l'extrémité proximale débouche dans la partie distale 2 de la poignée de commande 1.

Les divers dispositifs communément intégrés dans ce fibroscope, bien connus de l'homme du métier, n'ont volontairement pas été représentés, et ce pour des raisons de clarté, dans la figure 1. Il s'agit des éléments intégrés dans la partie distale de la gaine souple 3 (objectif, fenêtre d'illumination, sortie du canal opératoire, béquillage articulé ...), dans la gaine souple 3 (faisceau de fibres d'éclairage, faisceau ordonné de fibres image, câbles de commande du béquillage ...), dans la poignée de commande 1 (dispositif de commande du béquillage ...), dans la partie proximale 4 de la poignée (lentille oculaire, dispositif de réglage dioptrique commandé par la bague 5 ...), dans le câble ombilical souple 7 (faisceau de fibres d'éclairage ...) et dans l'extrémité proximale du câble ombilical (embout de raccordement à un générateur de lumière ...).

La partie distale 2 de la poignée de commande 1 du fibroscope illustrée par la figure 1 dispose d'une première embase latérale oblique 40 dans laquelle est ménagé un canal cylindrique 41 dont l'extrémité proximale 42 de forme conique constitue l'entrée instrumentation du fibroscope et dont l'extrémité distale C est reliée à l'extrémité proximale du canal opératoire 9 par une tubulure cylindrique rigide 12 fixement logée dans la partie distale 2 de la poignée de commande. L'extrémité proximale de l'embase 40 dispose d'un épaulement externe 43 permettant la mise en place d'un capuchon d'étanchéité 20 en matière plastique souple. Le capuchon dispose d'un orifice circulaire de passage 21 susceptible d'être occulté par un opercule cylindrique 22 solidaire d'un couvercle d'étanchéité 23 lié de façon élastique au capuchon.

La poignée de commande 1 du fibroscope illustré par la figure 1 dispose également d'une seconde embase latérale 44 dans laquelle est ménagé un canal cylindrique 45 dont l'extrémité proximale constitue l'entrée d'aspiration du fibroscope sur laquelle est connecté l'ensemble d'aspiration F et dont l'extrémité distale est reliée à l'extrémité distale de la tubulure 12 par une tubulure cylindrique rigide 13 fixement logée dans la partie distale 2 de la poignée de commande. La partie proximale de l'embase 44 dispose d'un filetage externe 46 destiné à la connexion de l'ensemble d'aspiration F d'un dispositif d'exploitation externe amovible.

Le dispositif d'exploitation comprend donc un ensemble d'aspiration F comportant un tube 55 dont l'extrémité distale dispose d'un orifice circulaire axial 56 et dont la partie proximale abrite un canal cylindrique axial 57. Le tube 55 de l'ensemble d'aspiration F est fixement solidaire d'une tubulure d'aspiration latérale rigide 10 dont l'extrémité distale débouche dans le canal cylindrique 57 aussi appelé conduit d'aspiration. L'extrémité distale du tube 55 est solidaire d'une bague folle 59 dont la partie distale dispose d'un filetage interne permettant de fixer le dispositif d'exploitation sur la poignée de commande 1 en vissant la bague sur le filetage externe 46 ménagé autour de la seconde embase 44. La partie proximale du tube 55 dispose d'un filetage externe 58 permettant de fixer sur ledit tube, le mécanisme de robinet 30 décrit ci-après.

Ce mécanisme de robinet démontable 30 est constitué d'une pièce cylindrique 31 logée de façon coulissante dans le conduit d'aspiration 57 ménagé dans la partie proximale du tube 55 du dispositif d'exploitation. La partie distale de la pièce cylindrique 31 dispose d'un canal axial borgne 34 généralement cylindrique et d'un orifice circulaire latéral 35 débouchant dans l'extrémité proximale du canal borgne 34. La partie proximale de la pièce cylindrique 31 dispose quant à elle d'une extrémité proximale 33 en forme de bouton poussoir, d'un épaulement circulaire externe 32 et d'une bague folle 37 dont la tranche proximale repose sur la face proximale de l'épaulement 32 et dont la partie distale présente un filetage interne permettant de visser la bague 37 sur le filetage externe 46 ménagé autour de la partie proximale du tube 55 du dispositif d'exploitation. Un ressort hélicoïdal 36 logé entre la tranche proximale du tube 55 et la face distale de l'épaulement 32 maintient le mécanisme de robinet 30 dans une position de repos caractérisée par une occultation de d'aspiration 10. Une action manuelle sur l'extrémité proximale 33 du mécanisme de robinet venant mettre en coïncidence l'orifice latéral 35 et l'extrémité distale du tube d'aspiration 10, et permettant donc de relier le tube d'aspiration 10 au canal 45 ménagé dans l'embase 44.

La figure 2 illustre la structure d'une poignée 1 de fibroscope à vocation médicale disposant d'un seul et unique accès 48 au canal opératoire 9 en position proximale et d'un dispositif d'exploitation E1 externe représentatif de l'état de l'art en la matière.

Comme toutes les sondes endoscopiques souples à vocation médicale équipées d'un canal opératoire 9, ce fibroscope dispose d'une poignée de commande 1 solidaire de l'extrémité distale d'un câble ombilical souple 7 et de l'extrémité proximale d'une gaine souple 3 servant notamment de logement au canal opératoire souple 9 dont l'extrémité proximale débouche dans la partie distale 2 de la poignée de commande 1.

Les divers dispositifs communément intégrés dans ce fibroscope, bien connus de l'homme du métier, n'ont volontairement pas été représentés sur la figure 2, et ce pour des raisons de clarté. Il s'agit des éléments intégrés dans la partie distale de la gaine souple 3 (objectif, fenêtre d'illumination, sortie du canal opératoire, béquillage articulé...), dans la gaine souple 3 (faisceau de fibres d'éclairage, faisceau ordonné de fibres image, câbles de commande du béquillage...), dans la poignée de commande 1 (dispositif de commande du béquillage...), dans la partie proximale 4 de la poignée (lentille oculaire, dispositif de réglage dioptrique commandé par la bague 5...), dans le câble ombilical souple 7 (faisceau de fibres d'éclairage...) et dans l'extrémité proximale du câble ombilical (embout de raccordement à un générateur de lumière...).

La poignée de commande 1 du fibroscope illustré par la figure 2 dispose d'une embase latérale oblique 47 dans laquelle est ménagé un canal cylindrique 48 dont l'extrémité distale est reliée à l'extrémité proximale du canal opératoire 9 par une tubulure cylindrique rigide 14 logée dans la partie distale 2 de la poignée de commande. La partie proximale de l'embase 47 dispose d'un filetage externe 49 destiné à la connexion d'un dispositif d'exploitation E1 externe.

Le dispositif d'exploitation E1 présente la forme d'un Y dont la branche principale, située dans le prolongement de l'embase 47, abrite le canal instrumentation et dont la branche latérale oblique abrite le canal aspiration. La branche principale du dispositif d'exploitation E1 est constituée d'un tube 60 dans lequel est ménagé un canal cylindrique 61 dont l'extrémité proximale 63 de forme conique constitue l'entrée instrumentation. L'extrémité proximale 63 du tube 60 dispose d'un épaulement externe 65 permettant la mise en place du capuchon d'étanchéité 20 décrit ci-avant en référence à la figure 1. L'extrémité distale C du tube 60 est solidaire d'une bague folle 64 dont la partie distale dispose d'un filetage interne permettant de fixer le dispositif d'exploitation E1 sur la poignée de commande 1 en vissant la bague 64 sur le filetage externe 49 ménagé autour de l'embase 47. La branche latérale oblique du dispositif d'exploitation E1 est constituée d'un tube 66 fixement solidaire du tube 60 et dont l'extrémité distale abrite un canal axial 67 débouchant dans l'extrémité distale 62 du canal 61 ménagé dans le tube 60. La partie proximale du tube 66 dispose d'un filetage externe 69 permettant de visser sur ledit tube la bague 37 du mécanisme de robinet 30 décrit ci-avant en référence à la figure 1 et préalablement introduit dans le canal cylindrique 68 ménagé dans ladite partie proximale. Le tube 66 est par ailleurs fixement solidaire d'une tubulure d'aspiration latérale rigide 10 dont l'extrémité distale débouche dans le canal cylindrique 68.

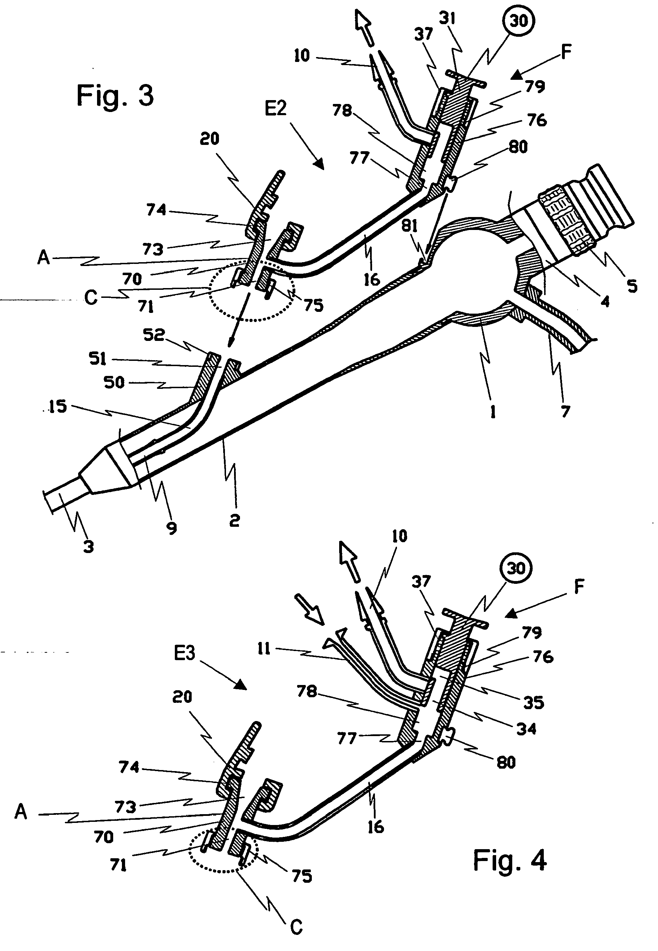

La figure 3 illustre la structure d'un fibroscope à vocation médicale disposant d'un seul et unique accès 51 au canal opératoire 9 et bénéficiant du dispositif opératoire externe faisant l'objet de la présente invention.

Comme toutes les sondes endoscopiques souples à vocation médicales équipées d'un canal opératoire, ce fibroscope dispose d'une poignée de commande 1 solidaire de l'extrémité distale d'un câble ombilical souple 7 et de l'extrémité proximale d'une gaine souple 3 servant notamment de logement au canal opératoire souple 9 dont l'extrémité proximale débouche dans la partie distale 2 de la poignée de commande 1.

Les divers dispositifs communément intégrés dans ce fibroscope, bien connus des spécialistes en la matière et ci-dessus évoqués, n'ont volontairement pas été représentés, et ce pour des raisons de clarté, dans la figure 3. Il s'agit des éléments intégrés dans la partie distale de la gaine souple 3 (objectif, fenêtre d'illumination, sortie du canal opératoire, béquillage articulé...), dans la gaine souple 3 (faisceau de fibres d'éclairage, faisceau ordonné de fibres image, câbles de commande du béquillage...), dans la poignée de commande 1 (dispositif de commande du béquillage...), dans la partie proximale 4 de la poignée (lentille oculaire, dispositif de réglage dioptrique commandé par la bague 5...), dans le câble ombilical souple 7 (faisceau de fibres d'éclairage...) et dans l'extrémité proximale du câble ombilical (embout de raccordement à un générateur de lumière...).

La partie distale 2 de la poignée de commande 1 du fibroscope illustré par la figure 3 dispose d'une embase latérale oblique 50 dans laquelle est ménagé un canal axial cylindrique 51 dont l'extrémité distale est reliée à l'extrémité proximale du canal opératoire 9 par une tubulure cylindrique rigide 15 fixement logée dans la partie distale 2 de la poignée de commande. La partie proximale de l'embase 50 dispose d'un filetage externe 52 destiné à la connexion du dispositif d'exploitation E2 externe faisant l'objet de la présente invention.

La partie distale du dispositif d'exploitation E2 comprend un moyen d'insertion A constituée d'une pièce cylindrique 70 dans laquelle est ménagé un conduit d'insertion d'instruments 71 dont l'extrémité proximale 73, de forme évasée, constitue l'entrée d'instrument. L'extrémité proximale 73 de la pièce cylindrique 70 dispose d'un épaulement externe 74 permettant la mise en place du capuchon d'étanchéité 20 décrit ci-avant en référence à la figure 1. La pièce cylindrique 70 est par ailleurs fixement solidaire d'un conduit de liaison latéral rigide 16 dont l'extrémité distale débouche dans le conduit d'insertion d'instruments 71. L'extrémité distale C de la pièce cylindrique 70 du moyen d'insertion A est solidaire d'une bague folle 75 dont la partie distale dispose d'un filetage interne permettant de fixer le dispositif d'exploitation E2 sur la partie distale 2 de la poignée de commande 1 en vissant la bague folle 75 sur le filetage externe 52 ménagé autour de la partie proximale de l'embase 50.

La partie proximale du dispositif d'exploitation E2 est constituée d'un tube 76 dont l'extrémité distale dispose d'un orifice circulaire 77 dans lequel est fixement logée l'extrémité proximale du conduit de liaison rigide 16. La partie proximale du tube 76 dispose d'un filetage externe 79 permettant de visser sur ledit tube la bague 37 du mécanisme de robinet 30 décrit ci-avant en référence à la figure 1 et préalablement introduit dans le conduit d'aspiration 78 cylindrique ménagé dans la partie proximale. Le tube 76 est par ailleurs fixement solidaire d'une tubulure d'aspiration latérale rigide 10 dont l'extrémité distale débouche dans le conduit d'aspiration 78 de forme cylindrique. La partie distale du tube 76 dispose d'un doigt latéral externe 80 présentant un épaulement qui, introduit dans une fente ménagée à cet effet sur la poignée de commande 1, permet de solidariser la partie distale du dispositif d'exploitation E2 et la poignée de commande 1.

La figure 4 illustre une autre version du dispositif d'exploitation E3 faisant l'objet de la présente invention, version permettant de gérer simultanément avec le même mécanisme de robinet les fonctions d'aspiration et d'injection dans le canal opératoire 9 d'un liquide contenu dans une poche de perfusion reliée par un tuyau flexible au dispositif d'exploitation E3. La mise en oeuvre en bronchoscopie d'un tel dispositif permet d'effectuer des opérations dites de "lavage alvéolaire".

Le dispositif d'exploitation E3 illustré par la figure 4 ne diffère de celui décrit en référence à la figure 3 que par la structure de sa partie proximale constituée d'un tube 76 fixement solidaire d'une part d'une tubulure d'aspiration latérale rigide 10 dont l'extrémité proximale débouche dans la partie proximale du conduit d'aspiration 78 en forme de canal cylindrique, ménagé dans le tube 76 et d'autre part d'une tubulure d'injection latérale rigide 11 dont l'extrémité proximale débouche dans le conduit d'aspiration 78.

Quand le mécanisme de robinet 30 est au repos un tel dispositif d'exploitation E3 interdit toute communication entre le tube d'aspiration 10 et le conduit de liaison 16, et interdit également toute communication entre le tube d'injection de liquide 11 et le conduit de liaison 16.

Quand le mécanisme de robinet 30 est actionné par l'utilisateur, par coulissement dans une première position d'ouverture de la pièce cylindrique coulissante 31, le dispositif autorise la communication entre le tube d'aspiration 10 et le conduit de liaison 16 et interdit toute communication entre le tube d'injection de liquide 11 et le conduit de liaison 16.

Quand le mécanisme de robinet 30 est actionné par l'utilisateur, par coulissement dans une seconde position d'ouverture de la pièce cylindrique coulissante 31, le dispositif interdit la communication entre le tube d'aspiration 10 et le conduit de liaison 16 et autorise la communication entre le tube d'injection de liquide 11 et le conduit de liaison 16.

On constate donc, sous réserve bien sûr que l'entrée instrumentation 73 soit occultée par le capuchon d'étanchéité 20, qu'un tel dispositif d'exploitation E3 permet effectivement à l'utilisateur de gérer simultanément à l'aide d'un seul moyen de commande 30, une aspiration de fluide et/ou une injection de liquide dans le canal opératoire 9.

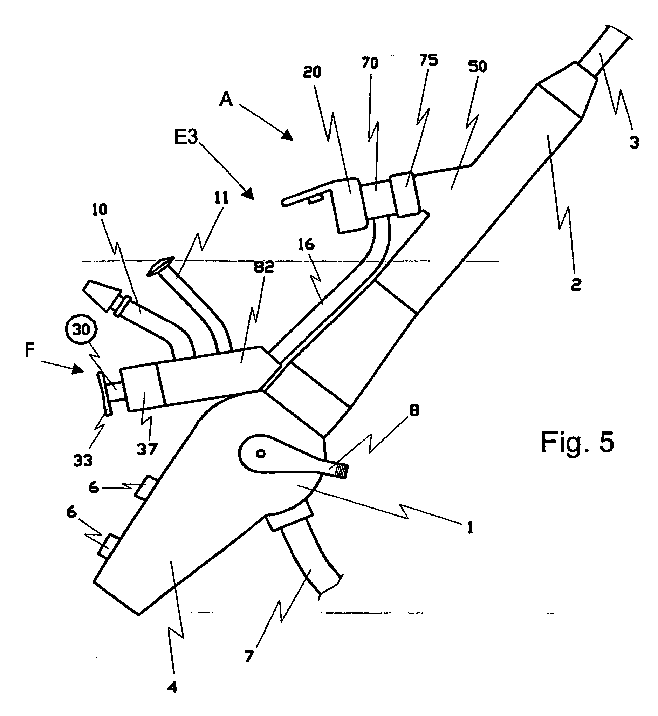

La figure 5 illustre l'aspect externe de la poignée de commande 1 d'une sonde vidéoendoscopique équipée du dispositif d'exploitation E3 ci-dessus décrit en référence à la figure 4.

La partie distale 2 de la poignée de commande dispose d'une embase 50 logeant le canal d'accès du canal opératoire et est fixement solidaire de l'extrémité proximale de la gaine souple 3 du vidéoendoscope, gaine dans l'extrémité distale de laquelle sont intégrés divers éléments (tels que béquillage articulé, fenêtre d'illumination, sortie du canal opératoire, dispositif optoélectronique...) bien connus de l'homme du métier et ci-dessus évoqués. La poignée de commande 1 est quant à elle solidaire de l'extrémité distale d'un câble ombilical 7, câble dans l'extrémité proximale duquel sont intégrés divers éléments (tels qu'embout de connexion à un générateur d'éclairage, prise de connexion à un processeur vidéo...) également bien connus des spécialistes en la matière et également évoqués ci-dessus.

La poignée de commande 1 dispose d'un levier de commande 8 permettant de commander l'orientation du béquillage distal de la sonde vidéoendoscopique et d'une partie proximale 4 équipée de touches 6 permettant de télécommander certaines fonctions du processeur vidéo connecté sur l'extrémité proximale du câble ombilical 7.

Le dispositif d'exploitation E3 externe décrit ci-avant en référence à la figure 4 est fixé sur la poignée de commande 1 en vissant sur l'embase 50 la bague folle 75 solidaire de l'extrémité distale 70 du dispositif.

La partie proximale 82 du dispositif d'exploitation E3 dispose d'une entrée d'aspiration d'air 10, d'une entrée d'injection de liquide 11, et d'un mécanisme de robinet 30 fixé sur la partie proximale 32 en vissant sur la partie proximale la bague 37 solidaire du mécanisme de robinet 30.

Le conduit de liaison 16 relie l'extrémité distale 70 du dispositif d'exploitation E3, extrémité distale équipée de la bague étanche 20 décrite ci-dessus en référence à la figure 1, à l'extrémité proximale 82 du dispositif.

## Revendications

1. Sonde endoscopique comprenant une poignée de commande (1) et un dispositif d'exploitation (E2) conçu pour se fixer de façon amovible sur la poignée (1), cette poignée présentant une partie distale et une partie proximale et étant munie d'un canal opératoire interne (9) dont l'entrée débouche au niveau d'une partie distale de la poignée (1), le dispositif d'exploitation comprenant :
- un moyen d'insertion (A) pour l'insertion d'instruments opératoires, comportant un conduit d'insertion d'instruments (71), dont l'extrémité distale (C) comprend des moyens de connexion pour connecter le conduit d'insertion d'instruments à l'entrée du canal opératoire (9), et dont l'extrémité proximale (63) constitue une entrée d'instrument du dispositif d'exploitation et
- un ensemble d'aspiration (F) comprenant un conduit d'aspiration (78) communiquant avec le conduit d'insertion et un moyen de commande (30) pour relier sélectivement le conduit d'aspiration (78) à une pompe, pour aspirer des fluides à travers le canal opératoire (9),
- un conduit de liaison (16) mettant en communication le conduit d'aspiration (78) avec le conduit d'insertion (71), et s'étendant à l'extérieur de la poignée, **caractérisée en ce que**
* la poignée (1) comprend des moyens de commande (5, 6) situés sur la partie proximale de la poignée,
* et **en ce que** cette sonde comprend un moyen de fixation supplémentaire (80) situé sur l'ensemble d'aspiration (F), conçu pour coopérer avec des moyens de fixation complémentaires (81) prévus sur la partie proximale de la poignée (1).

2. Sonde endoscopique selon la revendication 1, **caractérisée en ce que** la longueur du conduit de liaison (16) est de l'ordre de l'épaisseur de plusieurs doigts d'une main.

3. Sonde endoscopique selon l'une quelconque des revendications 1 ou 2, **caractérisée en ce que** des moyens de connexion (75) du conduit d'insertion (71) sont conçus pour assurer la fixation amovible du dispositif d'exploitation sur la poignée (1).

4. Sonde endoscopique selon l'une quelconque des revendications 1 à 3, **caractérisée en ce que** les moyens de fixation (80, 81) consistent, sur le dispositif d'exploitation, en un doigt latéral externe (80) présentant un épaulement qui, introduit dans une fente (81) ménagée à cet effet sur la poignée de commande, permet de solidariser le dispositif d'exploitation et la poignée de commande.

5. Sonde endoscopique selon l'une quelconque des revendications 1 à 4, **caractérisée en ce que** le conduit de liaison (16) est constitué en matériau rigide.

6. Sonde endoscopique selon l'une quelconque des revendications 1 à 5, **caractérisée en ce que** le moyen de commande d'aspiration (30) est un robinet démontable (30) pouvant adopter au moins une configuration d'ouverture autorisant le passage de fluide entre le canal opératoire (9) et une tubulure d'aspiration (10) elle-même reliée à la pompe d'aspiration, et une configuration de fermeture interdisant le passage de fluide vers la tubulure d'aspiration (10).

7. Sonde endoscopique selon la revendication 6, **caractérisée en ce que** l'ensemble d'aspiration (F) comporte une pièce cylindrique (31) montée coulissante à l'intérieur du conduit d'aspiration (78), et comprenant un canal axial borgne (34) et un orifice latéral (35) reliant l'extérieur de la pièce cylindrique (31) à l'intérieur du canal cylindrique axial borgne (34), la pièce cylindrique obturant en configuration de fermeture, l'entrée de la tubulure d'aspiration, l'orifice latéral (35) étant en configuration d'ouverture, en regard de l'entrée de la tubulure d'aspiration (10).

8. Sonde endoscopique selon la revendication 7, **caractérisée en ce que** 1a pièce cylindrique (31) est ramenée en configuration de fermeture par des moyens de rappel élastiques (36).

9. Sonde endoscopique selon l'une quelconque des revendications 1 à 8, **caractérisée en ce que** le conduit d'aspiration (78) de l'ensemble d'aspiration (F) du dispositif d'exploitation (E3) est relié sélectivement par le moyen de commande (30) à une tubulure d'injection (11) permettant l'injection sélective de fluides provenant de la tubulure d'injection (11) vers le canal opératoire (9).

10. Sonde endoscopique selon l'une quelconque des revendications 1 à 9, **caractérisée en ce que** le moyen d'insertion (A) pour l'insertion d'instruments opératoires est doté d'un capuchon (20) permettant l'obturation du conduit d'insertion (71).

11. Sonde endoscopique selon l'une quelconque des revendications 1 à 10, **caractérisée en ce que** le dispositif d'exploitation est réalisé essentiellement en matériau stérilisable à usage médical tel que de l'inox.

12. Sonde endoscopique selon l'une quelconque des revendications 1 à 11, **caractérisée en ce que** le dispositif d'exploitation est réalisé de manière à pouvoir être à usage unique.

13. Sonde endoscopique selon l'une quelconque des revendications 1 à 12, **caractérisée en ce que** la sonde est un fibroscope.

14. Sonde endoscopique selon l'une quelconque des revendications 1 à 13, **caractérisé en ce que** la sonde est un vidéoendoscope.

## Claims

1. Endoscopic probe comprising a control handle (1) and an application device (E2), the latter being designed to attach removably to the handle (1), this handle having a distal part and a proximal part and being provided with an internal operating canal (9) whose entrance is in a distal part of the handle (1), the application device comprising:
- an insertion means (A) for the insertion of operating instruments, comprising an instrument insertion tube (71) whose distal end (C) comprises connection means for connecting the instrument insertion tube to the entrance of the operating canal (9) and whose proximal end (63) forms an instrument entrance for the application device.
- a suction assembly (F) comprising a suction tube (78) communicating with the insertion tube and a control means (30) for selectively connecting the suction tube (78) to a pump to aspirate fluids through the operating canal (9), and
- a linking tube (16) connecting the suction tube (78) to the insertion tube (71) and extending along the outside of the handle, which probe is **characterized in that**
* the handle (1) comprises control means (5, 6) situated on the proximal part of the handle,
* and **in that** this probe comprises a supplementary attachment means (80) situated on the suction assembly (F) to engage with mating attachment means (81) on the proximal part of the handle (1).

2. Endoscopic probe according to Claim 1, **characterized in that** the length of the linking tube (16) is of the order of the thickness of several fingers of one hand.

3. Endoscopic probe according to either of Claims 1 and 2, **characterized in that** connection means (75) for the insertion tube (71) are designed to attach the application device removably to the handle (1).

4. Endoscopic probe according to any one of Claims 1 to 3, **characterized in that** the attachment means (80, 81) consist of, on the application device, an external lateral tooth (80) with a shoulder which, when inserted into a slot (81) formed for this purpose in the control handle, fixes the application device and the control handle to each other.

5. Endoscopic probe according to any one of Claims 1 to 4, **characterized in that** the linking tube (16) is made of a rigid material.

6. Endoscopic probe according to any one of Claims 1 to 5, **characterized in that** the suction control means (30) is a removable tap (30) able to adopt at least one open configuration in which fluid is allowed to pass between the operating canal (9) and a suction line (10), which in turn is connected to the suction pump, and a closed configuration in which fluid is not allowed to pass into the suction line (10).

7. Endoscopic probe according to Claim 6, **characterized in that** the suction assembly (F) comprises a cylindrical component (31) sliding inside the suction tube (78) and comprising a blind axial canal (34) and a lateral orifice (35) connecting the exterior of the cylindrical component (31) to the interior of the blind axial cylindrical canal (34), which cylindrical component, when in the closed configuration, blocks the entrance of the suction line, whereas when it is in the open configuration the lateral orifice (35) is positioned in front of the entrance of the suction line (10).

8. Endoscopic probe according to Claim 7, **characterized in that** the cylindrical component (31) is returned to the closed configuration by elastic return means (36).

9. Endoscopic probe according to any one of Claims 1 to 8, **characterized in that** the suction tube (78) of the suction assembly (F) of the application device (E3) is selectively connected by the control means (30) to an injection line (11) for the selective injection of fluids from the injection line (11) into the operating canal (9).

10. Endoscopic probe according to any one of Claims 1 to 9, **characterized in that** the insertion means (A) for the insertion of operating instruments is fitted with a cap (20) to allow the insertion tube (71) to be closed.

11. Endoscopic probe according to any one of Claims 1 to 10, **characterized in that** the application device is made essentially from a sterilizable medical-use material such as stainless steel.

12. Endoscopic probe according to any one of Claims 1 to 11, **characterized in that** the application device is produced in such a way that it can be used on a single-use basis.

13. Endoscopic probe according to any one of Claims 1 to 12, **characterized in that** the probe is a fibrescope.

14. Endoscopic probe according to any one of Claims 1 to 13, **characterized in that** the probe is a video endoscope.

## Patentansprüche

1. Endoskopiesonde mit einem Steuergriff (1) und einer Entnahmeeinrichtung (E2), die zur lösbaren Befestigung am Griff (1) vorgesehen ist, wobei dieser Griff einen distalen Bereich und einen proximalen Bereich aufweist und mit einem inneren Operationskanal (9) versehen ist, dessen Eingang an einem distalen Bereich des Griffes (1) mündet, wobei die Entnahmeeinrichtung umfasst:
- ein Einführmittel (A) zur Einführung von Operationsinstrumenten, mit einem Instrumenten-Einführkanal (71), dessen distales Ende (C) Anschlussmittel umfasst, um den Instrumenten-Einführkanal am Eingang des Operationskanals (9) anzuschließen, und dessen proximales Ende (63) einen Instrumenteneingang der Entnahmeeinrichtung bildet, und
- eine Saugeinheit (F) mit einem Saugkanal (78), der mit dem Einführkanal kommuniziert, und einer Steuereinrichtung (30), um wahlweise den Ansaugkanal (78) mit einer Pumpe zu verbinden, um Fluide durch den Operationskanal (9) hindurch anzusaugen,
- einen Verbindungskanal (16), der den Ansaugkanal (78) mit dem Einführkanal (71) in Kommunikation bringt und sich zum Äußeren des Griffes hin erstreckt, **dadurch gekennzeichnet, dass**
* der Griff (1) Steuermittel (5, 6) umfasst, die auf dem proximalen Bereich des Griffs liegen,
* und dadurch, dass die Sonde ein zusätzliches Befestigungsmittel (80) umfasst, das auf der Saugeinheit (F) angeordnet ist, um mit dem zusätzlichen Befestigungsmittel (81) kooperieren zu können, die auf dem proximalen Bereich des Griffes (1) vorgesehen sind.

2. Endoskopsonde nach Anspruch 1, **dadurch gekennzeichnet, dass** die Länge des Verbindungskanal (16) in etwa die Dicke mehrerer Daumen einer Hand beträgt.

3. Endoskopsonde nach einem der Ansprüche 1 oder 2, **dadurch gekennzeichnet, dass** das Anschlussmittel (75) des Einführkanals (71) vorgesehen ist, um die lösbare Befestigung der Entnahmeeinrichtung am Griff (1) zu gewähren.

4. Endoskopssonde nach einem der Ansprüche 1 bis 3, **dadurch gekennzeichnet, dass** die Befestigungsmittel (80, 81) auf der Entnahmeeinrichtung aus einem äußeren Seitenfinger (80) bestehen, der eine Schulter aufweist, die, wenn sie in einen Schlitz 81 eingeführt ist, der zu diesem Zweck auf dem Steuergriff ausgebildet ist, ermöglicht, die Entnahmeeinrichtung und den Steuergriff fest miteinander zu verbinden.

5. Endoskopsonde nach einem der Ansprüche 1 bis 4, **dadurch gekennzeichnet, dass** der Verbindungskanal (16) aus steifem Material gebildet ist.

6. Endoskopsonde nach einem der Ansprüche 1 bis 5, **dadurch gekennzeichnet, dass** die Saug-Steuereinrichtung (30) ein abnehmbares Ventil (30) ist, das wenigstens eine Öffnungsstellung annehmen kann, die den Fluiddurchgang zwischen dem Operationskanal (9) und einem Saugrohr (10) zulässt, das selbst mit der Saugpumpe verbunden ist, und eine Schließstellung annehmen kann, die den Fluiddurchgang zum Saugrohr (10) unterbindet.

7. Endoskopsonde nach Anspruch 6, **dadurch gekennzeichnet, dass** die Saugeinheit (F) ein Zylinderstück (31) umfasst, das verschiebbar im Inneren des Saugkanals (78) montiert ist, und einen axialen Blindkanal (34) und eine Seitenöffnung (35) umfasst, die das Äußere des Zylinderstückes (31) mit dem Inneren des zylinderförmigen axialen Blindkanals (34) verbindet, wobei das Zylinderstück in Schließstellung den Eingang des Saugrohrs verschließt, wobei die Seitenöffnung (35) in Bezug zu dem Eingang des Saugrohrs (10) in Öffnungsstellung ist.

8. Endoskopsonde nach Anspruch 7, **dadurch gekennzeichnet, dass** das Zylinderstück (31) durch ein elastisches Rückstellmittel (36) in Schließstellung zurückgebracht wird.

9. Endoskopsonde nach einem der Ansprüche 1 bis 8, **dadurch gekennzeichnet, dass** der Saugkanal (78) der Saugeinheit (F) der Entnahmeeinrichtung (E3) wahlweise durch die Steuereinrichtung (30) mit einem Injektionsrohr (11) verbunden ist, das die wahlfreie Injektion von Fluiden aus dem Injektionsrohr (11) in den Operationskanal (9) ermöglicht.

10. Endoskopsonde nach einem der Ansprüche 1 bis 9, **dadurch gekennzeichnet, dass** die Einführeinrichtung (A) für die Einführung von Operationsinstrumenten mit einer Kappe (20) versehen ist, welche den Verschluss des Einführkanals (71) ermöglicht.

11. Endoskopsonde nach einem der Ansprüche 1 bis 10, **dadurch gekennzeichnet, dass** die Entnahmeeinrichtung im Wesentlichen aus sterilisierbarem Material zur medizinischen Verwendung, wie Inox, hergestellt ist.

12. Endoskopsonde nach einem der Ansprüche 1 bis 11, **dadurch gekennzeichnet, dass** die Entnahmeeinrichtung so hergestellt ist, dass sie einmal-verwendbar ist.

13. Endoskopsonde nach einem der Ansprüche 1 bis 12, **dadurch gekennzeichnet, dass** die Sonde ein Fiberendoskop ist.

14. Endoskopsonde nach einem der Ansprüche 1 bis 13, **dadurch gekennzeichnet, dass** die Sonde ein Videoendoskop ist.
